# EUROPEAN PATENT APPLICATION

(11) **EP 1 336 652 A1**
(43) Date of publication of application: **20.08.2003**
(21) Application number: 01997191.0
(22) Date of filing: 22.11.2001
(51) Int. Cl.: C12N 5/10, C12N 15/86, C12N 7/01

(54) **CELLS TO BE USED IN PRODUCING VIRUS VECTOR, PROCESS FOR PRODUCING THE SAME AND PROCESS FOR PRODUCING VIRUS VECTOR WITH THE USE OF THE CELLS**

(30) Priority: 22.11.2000 JP 2000355850
(71) Applicant: HISAMITSU PHARMACEUTICAL CO. INC., Tosu-shi, Saga-ken 841-0017 (JP)
(72) Inventor: SHIMADA, Takashi, Bunkyo-ku, Tokyo 113-0023 (JP)
(74) Representative: Grund, Martin, Dr.
(86) International application number: JP0110213
(87) International publication number: WO02042434

(57) **Abstract**

It is intended to establish a novel cell line for efficiently producing a virus vector without resort to any troublesome operations and provide a process for producing a virus vector having a high titer with the use of the cell line. Namely, cells to be used in producing a virus vector having an antisense gene been transferred thereinto are provided, wherein the gene expresses an antisense RNA against the whole or partial sequence of a sense RNA expressed by a gene encoding a cytotoxic polypeptide.

SEQUENCE LISTING

<110> Hisamitsu Pharmaceutical Co., Inc.

<120> A CELL FOR PREPARING A VIRUS VECTOR, A METHOD FOR
PREPARING THEREOF, AND A METHOD FOR PREPARING A VIRUS
VECTOR USING THE CELL

<130> 7-35

<140> EP01997191.0

<141> 2001-11-22

<160> 6

<170> PatentIn Ver. 2.1

<210> 1

<211> 826

<212> DNA

<213> Artificial Sequence

<220>

<223> Description of Artificial Sequence:Antisense
sequence between p5 and p19 of rep gene

<400> 1

gaagatctga attcgccgca ttgaaggaga tgtatgaggc ctggtcctcc tggatccact 60
gcttctccga ggtaatcccc ttgtccacga gccacccgac cagctccatg tacctggctg 120
aagtttttga tctgatcacc ggcgcatcag aattgggatt ctgattctct ttgttctgct 180
cctgcgtctg cgacacgtgc gtcagatgct gcgccaccaa ccgtttacgc tccgtgagat 240
tcaaacaggc gcttaaatac tgttccatat tagtccacgc ccactggagc tcaggctggg 300
ttttggggag caagtaattg gggatgtagc actcatccac caccttgttc ccgcctccgg 360
cgccatttct ggtctttgtg accgcgaacc agtttggcaa agtcggctcg atcccgcggt 420
aaattctctg aatcagtttt tcgcgaatct gactcaggaa acgtcccaaa accatggatt 480
tcaccccggt ggtttccacg agcacgtgca tgtggaagta gctctctccc ttctcaaatt 540
gcacaaagaa aagggcctcc ggggccttac tcacacggcg ccattccgtc agaaagtcgc 600
gctgcagctt ctcggccacg gtcaggggtg cctgctcaat cagattcaga tccatgtcag 660
aatctggcgg caactcccat tccttctcgg ccacccagtt cacaaagctg tcagaaatgc 720
cgggcagatg cccgtcaagg tcgctgggga ccttaatcac aatctcgtaa aaccccggca 780
tggcggctgc gcgttcaaac ctcccgcttc aaaatggaag atcttc 826

<210> 2

<211> 35

<212> DNA
<213> Artificial Sequence
<220>
<223> Description of Artificial Sequence:Sence primer
for cloning
<400> 2
gaagatcttc cattttgaag cgggaggttt gaacg 35

<210> 3
<211> 39
<212> DNA
<213> Artificial Sequence
<220>
<223> Description of Artificial Sequence:Antisence
primer for cloning
<400> 3
gaagatctga attcgccgca ttgaaggaga tgtatgagg 39

<210> 4
<211> 631
<212> DNA
<213> Artificial Sequence
<220>
<223> Description of Artificial Sequence:XbaI-SacI
fragment for hybridization
<400> 4
tctagagtcc tgtattagag gtcacgtgag tgttttgcga cattttgcga caccatgtgg 60
tcacgctggg tatttaagcc cgagtgagca cgcagggtct ccattttgaa gcgggaggtt 120
tgaacgcgca gccgccatgc cggggtttta cgagattgtg attaaggtcc ccagcgacct 180
tgacgggcat ctgcccggca tttctgacag ctttgtgaac tgggtggccg agaaggaatg 240
ggagttgccg ccagattctg acatggatct gaatctgatt gagcaggcac ccctgaccgt 300
ggccgagaag ctgcagcgcg actttctgac ggaatggcgc cgtgtgagta aggccccgga 360
ggcccttttc tttgtgcaat ttgagaaggg agagagctac ttccacatgc acgtgctcgt 420
ggaaaccacc ggggtgaaat ccatggtttt gggacgtttc ctgagtcaga ttcgcgaaaa 480
actgattcag agaatttacc gcgggatcga gccgactttg ccaaactggt tcgcggtcac 540
aaagaccaga aatggcgccg gaggcgggaa caaggtggtg gatgagtgct acatccccaa 600

ttacttgctc cccaaaaccc agcctgagct c 631

<210> 5

<211> 1608

<212> DNA

<213> adeno associated virus

<220>

<223> rep gene encoding REP68 protein

<400> 5

atgccggggt tttacgagat tgtgattaag gtccccagcg accttgacgg gcatctgccc 60

ggcatttctg acagctttgt gaactgggtg gccgagaagg aatgggagtt gccgccagat 120

tctgacatgg atctgaatct gattgagcag gcacccctga ccgtggccga gaagctgcag 180

cgcgactttc tgacggaatg gcgccgtgtg agtaaggccc cggaggccct tttctttgtg 240

caatttgaga agggagagag ctacttccac atgcacgtgc tcgtggaaac caccggggtg 300

aaatccatgg ttttgggacg tttcctgagt cagattcgcg aaaaactgat tcagagaatt 360

taccgcggga tcgagccgac tttgccaaac tggttcgcgg tcacaaagac cagaaatggc 420

gccggaggcg ggaacaaggt ggtggatgag tgctacatcc ccaattactt gctccccaaa 480

acccagcctg agctccagtg ggcgtggact aatatggaac agtatttaag cgcctgtttg 540

aatctcacgg agcgtaaacg gttggtggcg cagcatctga cgcacgtgtc gcagacgcag 600

gagcagaaca aagagaatca gaatcccaat tctgatgcgc cggtgatcag atcaaaaact 660

tcagccaggt acatggagct ggtcgggtgg ctcgtggaca aggggattac ctcggagaag 720

cagtggatcc aggaggacca ggcctcatac atctccttca atgcggcctc caactcgcgg 780

tcccaaatca aggctgcctt ggacaatgcg ggaaagatta tgagcctgac taaaaccgcc 840

cccgactacc tggtgggcca gcagcccgtg gaggacattt ccagcaatcg gatttataaa 900

attttggaac taaacgggta cgatccccaa tatgcggctt ccgtctttct gggatgggcc 960

acgaaaaagt tcggcaagag gaacaccatc tggctgtttg ggcctgcaac taccgggaag 1020

accaacatcg cggaggccat agcccacact gtgcccttct acgggtgcgt aaactggacc 1080

aatgagaact ttcccttcaa cgactgtgtc gacaagatgg tgatctggtg ggaggagggg 1140

aagatgaccg ccaaggtcgt ggagtcggcc aaagccattc tcggaggaag caaggtgcgc 1200

gtggaccaga aatgcaagtc ctcggcccag atagacccga ctcccgtgat cgtcacctcc 1260

aacaccaaca tgtgcgccgt gattgacggg aactcaacga ccttcgaaca ccagcagccg 1320

ttgcaagacc ggatgttcaa atttgaactc acccgccgtc tggatcatga ctttgggaag 1380

gtcaccaagc aggaagtcaa agactttttc cggtgggcaa aggatcacgt ggttgaggtg 1440

gagcatgaat tctacgtcaa aaagggtgga gccaagaaaa gacccgcccc cagtgacgca 1500

gatataagtg agcccaaacg ggtgcgcgag tcagttgcgc agccatcgac gtcagacgcg 1560

gaagcttcga tcaactacgc agacagattg gctcgaggac actctctc 1608

<210> 6

<211> 1863

<212> DNA

<213> adeno associated virus

<220>

<223> rep gene encoding REP78 protein

<400> 6

atgccggggt tttacgagat tgtgattaag gtccccagcg accttgacgg gcatctgccc 60

ggcatttctg acagctttgt gaactgggtg gccgagaagg aatgggagtt gccgccagat 120

tctgacatgg atctgaatct gattgagcag gcacccctga ccgtggccga gaagctgcag 180

cgcgactttc tgacggaatg gcgccgtgtg agtaaggccc cggaggccct tttctttgtg 240

caatttgaga agggagagag ctacttccac atgcacgtgc tcgtggaaac caccggggtg 300

aaatccatgg ttttgggacg tttcctgagt cagattcgcg aaaaactgat tcagagaatt 360

taccgcggga tcgagccgac tttgccaaac tggttcgcgg tcacaaagac cagaaatggc 420

gccggaggcg ggaacaaggt ggtggatgag tgctacatcc ccaattactt gctccccaaa 480

acccagcctg agctccagtg ggcgtggact aatatggaac agtatttaag cgcctgtttg 540

aatctcacgg agcgtaaacg gttggtggcg cagcatctga cgcacgtgtc gcagacgcag 600

gagcagaaca aagagaatca gaatcccaat tctgatgcgc cggtgatcag atcaaaaact 660

tcagccaggt acatggagct ggtcgggtgg ctcgtggaca aggggattac ctcggagaag 720

cagtggatcc aggaggacca ggcctcatac atctccttca atgcggcctc caactcgcgg 780

tcccaaatca aggctgcctt ggacaatgcg ggaaagatta tgagcctgac taaaaccgcc 840

cccgactacc tggtgggcca gcagcccgtg gaggacattt ccagcaatcg gatttataaa 900

attttggaac taaacgggta cgatccccaa tatgcggctt ccgtctttct gggatgggcc 960

acgaaaaagt tcggcaagag gaacaccatc tggctgtttg ggcctgcaac taccgggaag 1020

accaacatcg cggaggccat agcccacact gtgcccttct acgggtgcgt aaactggacc 1080

aatgagaact ttcccttcaa cgactgtgtc gacaagatgg tgatctggtg ggaggagggg 1140

aagatgaccg ccaaggtcgt ggagtcggcc aaagccattc tcggaggaag caaggtgcgc 1200

gtggaccaga aatgcaagtc ctcggcccag atagacccga ctcccgtgat cgtcacctcc 1260

aacaccaaca tgtgcgccgt gattgacggg aactcaacga ccttcgaaca ccagcagccg 1320

ttgcaagacc ggatgttcaa atttgaactc acccgccgtc tggatcatga ctttgggaag 1380

gtcaccaagc aggaagtcaa agactttttc cggtgggcaa aggatcacgt ggttgaggtg 1440

gagcatgaat tctacgtcaa aaagggtgga gccaagaaaa gacccgcccc cagtgacgca 1500

gatataagtg agcccaaacg ggtgcgcgag tcagttgcgc agccatcgac gtcagacgcg 1560

gaagcttcga tcaactacgc agacaggtac caaaacaaat gttctcgtca cgtgggcatg 1620

aatctgatgc tgtttccctg cagacaatgc gagagaatga atcagaattc aaatatctgc 1680

ttcactcacg gacagaaaga ctgtttagag tgctttcccg tgtcagaatc tcaacccgtt 1740
tctgtcgtca aaaaggcgta tcagaaactg tgctacattc atcatatcat gggaaaggtg 1800
ccagacgctt gcactgcctg cgatctggtc aatgtggatt tggatgactg catctttgaa 1860
caa 1863

## Description

### Technical Field

The present invention relates to cells used in producing a virus vector and, in particular, a high titer virus vector, a process for producing the cells, and a process for producing a virus vector and, in particular, a high titer virus vector using the cells.

### Background Art

In recent years, methods employing virus vectors have become widely known as methods for transferring genes into animals, including humans, and as the virus vectors used therein are known virus vectors employing retrovirus, lentivirus, adenovirus, adeno-associated virus, etc.

An adeno-associated virus (AAV: Adeno-Associated Virus) vector can insert genomic DNA into a host chromosome DNA, but wild type AAV itself is nonpathogenic (N. Muzyczka, Current Topics in Microbiology and Immunology, 158, 97, 1992). The AAV vector has the characteristics of being capable of transferring a gene into nondividing cells such as hematopoietic stem cells, being capable of transferring a gene selectively into human chromosome 19, etc. (M. Suwadogo and R. G. Roder, Proc. Natl. Acad. Sci. U.S.A., 82, 4394, 1985). Furthermore, since AAV particles are physically stable, it is possible to prepare a vector having a high transduction efficiency by concentration employing sucrose gradient centrifugation, cellulose sulfate affinity chromatography, etc. (K. Tamayose, et al., Hum. Gene Ther., 7, 507-513, 1996).

AAV is a replication deficient virus (genus *Dependentvirus*) and requires the aid of a helper virus such as an adenovirus (Ad: Adenovirus) for proliferation. In the case of use of the AAV vector, preparation has been carried out by simultaneous infection with adenovirus, which is a helper virus, in addition to cotransfection with a helper plasmid and a vector plasmid (R. M. Kotin, Hum. Gene Ther. 5, 793, 1994). However, a transfection method, represented by the calcium phosphate method, has the problems that (1) there is a limit to the efficiency of plasmid transduction into cells and it is difficult to obtain a high titer virus vector that is required in the clinical field, (2) when transfection is carried out in several lots, the transduction efficiency varies between the lots, and stable supply of virus vectors having a fixed titer is impossible, (3) since transfection operations are complicated, it is difficult to prepare a large quantity of vector all at once, (4) in order to prepare a large quantity of AAV vector it is necessary to prepare a large quantity of plasmid for transfection, etc.

A packaging cell, in which a helper plasmid is inserted into genomic DNA of a virus vector producing cell, has been considered as a means for solving these problems. Since the plasmid-derived DNA is stably inserted into the genomic DNA in this cell line, it can be passed on to daughter cells during cell division, and a given amount of recombinant virus can be obtained stably by culturing at a required scale. In general, establishment of these packaging cell lines is carried out by transfecting cells for producing a virus vector with a helper plasmid containing a drug-resistant gene such as a neomycin-resistant gene or a hygromycin-resistant gene, and packaging cells having a gene derived from the plasmid inserted into the genomic DNA can be obtained by extended culturing of the cells in a culture medium containing an antibiotic. The AAV vector is prepared using 293 cells, which constantly express adenovirus E1A and E1B genes. The 293 cell is a cell line established by transformation of human embryo kidney cells using adenovirus type 5 (Ad5) E1 gene (E1A and E1B genes).

Adenovirus E1A protein induces transcription from AAV p5 promoter so as to express AAV REP protein. The REP protein is a protein necessary when the AAV genome is transduced selectively into human chromosome 19, and it is expected to direct the protein synthesis system of the cell so as to produce AAV particles. However, the REP protein has cytotoxicity and the property of suppressing cell growth. Furthermore, it is thought that adenovirus E1B and E4 are necessary for storing mRNA and that E2A and VA are necessary for splicing and translating mRNA (N. Muzyczka, Current Topics in Microbiology and Immunology, 158, 97, 1992).

In 293 cells, which constantly express the E1 gene of adenovirus type 5, AAV p5 promoter is activated in the absence of the adenovirus, and the REP protein is expressed. When the REP protein is constantly expressed, cell growth inhibition occurs and the cells therefore die. Because of this, it is difficult to produce AAV vector packaging cells using 293 cells. Furthermore, the REP protein has the action of inhibiting growth of the adenovirus, which is a helper virus, and in the cells in which a rep gene is constantly expressed the growth of the adenovirus is inhibited. In order to avoid the toxicity of the rep gene product (that is, the REP protein) toward cells and the growth inhibition of the adenovirus, which is a helper virus, various methods have been examined. A method has been considered in which an AAV vector is prepared by transferring a gene encoding rep and/or cap genes by a known method into a packaging cell established by transferring only a vector plasmid-derived gene sequence to be inserted into the AAV vector. This method for transferring a gene sequence encoding the rep and/or cap genes into a cell can be roughly divided into a method employing a recombinant virus and a method not employing one, but the method employing the adenovirus has a high transduction efficiency and is preferable when producing a higher titer AAV vector.

In the case where 293 cells are used as packaging cells, since these cells already contain the adenovirus E1 gene, when producing an AAV vector, infecting only with an E1-deficient adenovirus instead of the wild type adenovirus can produce the AAV vector. Therefore, a method has been considered in which REP and/or CAP proteins are supplied by means of an adenovirus in which a gene sequence encoding the AAV rep and/or cap genes is inserted in the E1-deficient region. However, when an attempt was made to prepare the adenovirus by a known COS-TPC method (Kanegae, et al., Jikken Igaku, Vol. 12, No. 3, 1994, S. Miyake, et al., Proc. Natl. Acad. Sci. U.S.A., 93, 1320, 1996, JP, A, 8-84589, JP, A, 7-298877), since the REP protein was expressed in the 293 cells, an adenovirus containing the rep gene within its genomic sequence could not be obtained.

JP, A, 10-33175 discloses a method for producing a recombinant AAV vector in 293 cells by controlling expression of the rep gene using a Cre/loxP expression control system. However, even when employing this method, the Cre/loxP expression control system cannot completely control the expression of the rep gene. When an AAV vector is produced there are the problems that it is necessary to supply Cre and cut out loxP, the operations are complicated, the supply of Cre is also very difficult to control, and the expression control becomes unstable.

As hereinbefore described, since the conventional methods require complicated operations, plasmid transduction into cells is not uniform, plasmid DNA remains behind, the cost is high, etc., there has been a desire for an efficient process for producing a desired AAV vector.

### Disclosure of Invention

It is therefore an object of the present invention to establish a novel cell line for efficient production of a virus vector without complicated operations, and to provide a process for producing a high titer virus vector using this cell line.

As a result of an intensive investigation by the present inventors in order to solve the above-mentioned problems, a novel cell line for use in the production of a virus vector has been established, a process for producing a high titer virus vector using this cell line has been found, and the present invention has thus been accomplished.

That is, the present invention relates to cells to be used in producing a virus vector, the cells having been transferred thereinto one or two or more antisense genes that express an antisense RNA complementary to the entire sequence or a partial sequence of a sense RNA that is expressed by a gene encoding a polypeptide having cytotoxicity.

Furthermore, the present invention relates to the above-mentioned cells wherein the gene encoding the polypeptide having cytotoxicity is a virus vector-derived gene.

Moreover, the present invention relates to the above-mentioned cells wherein the virus vector-derived gene is an adeno-associated virus vector-derived gene.

Furthermore, the present invention relates to the above-mentioned cells wherein the adeno-associated virus vector-derived gene is a rep gene.

Moreover, the present invention relates to the above-mentioned cells wherein the antisense gene is an antisense gene that expresses an antisense RNA complementary to a sequence represented by SEQ ID NO: 1 and/or a sequence that is obtained by partially deleting, substituting, or adding to said sequence.

Furthermore, the present invention relates to the above-mentioned cells wherein the cells are cells designated by Depository No. FERM BP-7377.

Moreover, the present invention relates to the above-mentioned cells wherein the polypeptide having cytotoxicity is a polypeptide that inhibits growth of a helper virus.

Furthermore, the present invention relates to the above-mentioned cells wherein the helper virus is an adenovirus.

Moreover, the present invention relates to a process for producing cells to be used in producing a virus vector, comprising transferring one or two or more antisense genes that express an antisense RNA complementary to the entire sequence or a partial sequence of a sense RNA that is expressed by a gene encoding a polypeptide having cytotoxicity.

Furthermore, the present invention relates to the above-mentioned process wherein the gene encoding the polypeptide having cytotoxicity is a virus vector-derived gene.

Moreover, the present invention relates to the above-mentioned process wherein the virus vector-derived gene is an adeno-associated virus vector-derived gene.

Furthermore, the present invention relates to the above-mentioned process wherein the adeno-associated virus vector-derived gene is a rep gene.

Moreover, the present invention relates to the above-mentioned process wherein the antisense gene is an antisense gene that expresses an antisense RNA complementary to the sequence represented by SEQ ID NO: 1 and/or a sequence that is obtained by partially deleting, substituting, or adding to said sequence.

Furthermore, the present invention relates to the above-mentioned process wherein the cells are cells designated by Depository No. FERM BP-7377.

Moreover, the present invention relates to the above-mentioned process wherein the polypeptide having cytotoxicity is a polypeptide that inhibits growth of a helper virus.

Furthermore, the present invention relates to the above-mentioned process wherein the helper virus is an adenovirus.

Moreover, the present invention relates to a process for producing a virus vector with the use of the above-mentioned cells, comprising:
a step of obtaining a helper virus that expresses a gene derived from the virus vector; and
a step of transfecting cells into which no antisense gene has been transferred with the helper virus that expresses the gene derived from the virus vector and a virus vector plasmid.

In accordance with the present invention, even in the case where, in order to produce a target virus vector, a gene encoding a polypeptide having cytotoxicity is transferred into cells used in producing the virus vector and there is a possibility that it might be expressed within the cells, since an antisense gene is constantly expressed within the cells, expression of the gene encoding the polypeptide having cytotoxicity is suppressed.

That is, the cells of the present invention themselves can suppress expression of the gene encoding the polypeptide having cytotoxicity, and it is unnecessary to insert a special sequence into the gene encoding the polypeptide having cytotoxicity, unlike the case where the conventional Cre/loxP expression control system is employed; furthermore, it is unnecessary to give a special enzyme to the cells in order to control the expression of the gene, and the target virus vector can be produced efficiently by simple operations.

The cells designated by Depository No. FERM BP-7377 are cells deposited with the Patent Microorganism Depository Center of the National Institute of Advanced Industrial Science and Technology (1-3 Higashi 1-chome, Tsukuba-shi, Ibaraki, Japan 305-8566) on November 21, 2000.

### Brief Description of the Drawings

FIG. 1 is a photograph showing the results of Northern hybridization confirming the presence of cells expressing rep antisense of the present invention.

FIG. 2 is a graph showing a comparison of the transduction efficiency of a recombinant AAV vector solution prepared using an AD/AAV solution of the present invention and a recombinant AAV vector solution prepared using a conventional plasmid.

FIG. 3 is a schematic diagram showing the constitution of pCAGS/LN plasmid.

FIG. 4 is a schematic diagram showing the constitution of pCAGS-r/anti-p5+p19/LN plasmid.

FIG. 5 is a schematic diagram showing the constitution of pAAV/Ad plasmid.

FIG. 6 is a schematic diagram showing the constitution of pAdexlw cosmid.

FIG. 7 is a schematic diagram showing the constitution of pAdex1w/AAV cosmid.

FIG. 8 is a schematic diagram showing the constitution of COS-TPC in which the pAdex1w/AAV is treated with EcoT22I.

FIG. 9 is a schematic diagram showing the constitution of pCAGSETN/sub recombinant AAV vector vector plasmid.

FIG. 10 is a base sequence of a PCR amplification product (p5-19/PCR) transferred into the cells that express rep antisense of the present invention.

### Modes for Carrying Out the Invention

The 'virus vector' referred to in the present specification denotes one obtained by modifying a virus present in nature by a recombinant DNA technique so as to insert a given gene into the genome of the virus. Virus vectors can be roughly divided into DNA virus vectors in which the virus genome is DNA, and RNA virus vectors in which the virus genome is RNA. Virus vectors have the function of introducing and expressing a given gene into a target cell by utilizing the inherent transduction activity of the virus. Examples of the virus vector include an adeno-associated virus (AAV) vector, an adenovirus vector, a murine leukemia virus (MoMLV) vector, a human immunodeficiency virus (HIV) vector, a simian immunodeficiency virus (SIV) vector, a Sendai virus vector, and a herpesvirus vector. It is also possible to use in the present invention a pseudotype virus vector in which at least one of the structural protein groups of a virus vector is substituted by a structural protein of a different type of virus, or in which a part of a nucleic acid sequence forming genetic information is substituted by a nucleic acid sequence of a different type of virus.

The high titer virus vector denotes a virus vector having a high titer, that is, a virus vector having high transduction efficiency. The titer of a virus vector is usually denoted by CFU (colony forming units). It is evaluated by, for example, a method in which a virus vector obtained by insertion or substitution of a neomycin-resistant gene into virus genome is transferred into a given cell, and the number of colonies of the cells that grow in a neomycin-containing culture medium is counted.

The 'helper virus' referred to in the present specification denotes a virus that is required to induce replication of a virus vector that cannot replicate on its own. For example, AAV is a replication deficient virus and requires either adenovirus or herpesvirus for its replication. Such adenovirus and herpesvirus are helper viruses for the AAV.

The 'helper plasmid' referred to in the present specification denotes, for example, a plasmid that cuts a structural gene between the 5'-ITR sequence and the 3'-ITR sequence of the wild type AAV genome, that itself is not packaged, and that contains a gene sequence enabling expression of a protein necessary for producing the AAV vector. The helper plasmid can be constructed by a known method. The wild type AAV ITR (inverted terminal repeat) referred to here denotes a sequence of 145 bases present at both terminals of the AAV genomic DNA, has a T type hairpin structure, and is essential to replication, packaging, insertion into a chromosome, etc. of the virus (R. Samulski, et al., Proc. Natl. Acad. Sci. U.S.A. 79, 2077, 1982). This helper plasmid does not have a promoter sequence, and the AAV gene is expressed only by an AAV-derived promoter. The helper plasmid used for the production of an AAV vector enables transcription from p5 by using 293 cells as the packaging cells. The p5 referred to here is an AAV promoter and is usually present upstream of the rep gene.

The 'vector plasmid' referred to in the present specification is a plasmid having a target foreign gene for transfer (gene for transfer) and denotes, for example, an AAV vector plasmid in which a wild type AAV genomic sequence between the 5'-ITR sequence and the 3'-ITR sequence of the plasmid genome encoding the entire genomic sequence of a wild type AAV, represented by psub201, is substituted by at least one marker gene and/or a gene for transfer. The gene for transfer contains at least one promoter and a poly(A) signal, and examples of the promoter include a promoter derived from a virus such as adenovirus (Ad), cytomegalovirus (CMV), human immunodeficiency virus (HIV), adeno-associated virus (AAV), simian virus 40 (SV40), Rous sarcoma virus (RSV), herpes simplex virus (HSV), murine leukemia virus (MoMLV), Sindbis virus (Sindbis virus), Sendai virus (SeV), hepatitis A virus (HAV), hepatitis B virus (HBV), hepatitis C virus (HCV), human papilloma virus (HPV), bovine papilloma virus (BPV), human T-cell leukemia virus (HTLV), vesicular stomatitis virus (VSV), influenza virus (Influenza virus), Japanese encephalitis virus (Japanese encephalitis virus), JC virus (JC virus), Parvovirus B19 (Parvovirus B19), or poliovirus (Poliovirus); a mammalian-derived promoter such as SR-α (alpha subunit of signal recognition particle receptor), Myelin basic protein (MBP), glial specific glial fibrillary acidic protein (GFAP), β-actin, elongation factor-α (EF 1-α), glyceraldehyde-3-phosphate dehydrogenase (GAPDH), multidrug resistant gene (Mdr1), albumin alpha feto protein (AFP), heat shock protein (HSP), or hypoxia inducible protein (HIP); and a chimeric promoter such as a chimeric promoter (CAG) formed from CMV immediate-early enhancer/chicken β-actin promoter/β-globin poly(A), or a chimeric promoter formed from a CMV immediate-early enhancer/alpha-skeleton actin promoter. There can also be cited as examples a chimeric LTR in which the U3 region of LTR, which is a retrovirus-derived promoter for gene expression, is substituted by a promoter such as CAG, CMV, RSV, herpes simplex virus-thymidine kinase (HSV-TK), SV40, SR-α, MBP, β-actin, or EF1-α, etc., but the CAG promoter is preferable because, for example, the gene expression efficiency is high and the promoter has no host and organ specificity so that the gene can be expressed efficiently in various organs of various laboratory animals and humans. It is also possible to insert a drug resistant gene such as a neomycin-resistant gene for selection.

The 'packaging cells' referred to in the present specification are cells into which, in order to produce virus particles having infectivity, a gene encoding a required protein is transferred in advance by, for example, inserting it into a chromosome, and the virus particles produced in the packaging cells contain no virus genome. Supplying a virus genome into which a given gene has been inserted and/or substituted to the packaging cells can insert this virus genome into the virus particles, thus producing a virus vector. Examples of the cells used for the packaging cells include 293 cells, HeLa cells, COS cells, and 3T3 cells. When producing an AAV vector, a helper virus is needed, and the 293 cells are therefore preferable since they are easily infected by an adenovirus, which is a helper virus.

The 'polypeptide having cytotoxicity' referred to in the present specification denotes a polypeptide containing a protein that, when produced within cells for producing a virus vector, prevents the cells from maintaining their normal state due to cytotoxicity. Examples thereof include a REP protein encoded by a rep gene, a virus surface protein (ENV) encoded by the env gene of HIV, and a virus surface protein (VSV) encoded by the G gene of VSV.

The 'rep gene' referred to in the present description is an AAV-derived gene, and is a gene encoding a REP protein involved in AAV replication. The rep gene codes for four proteins, that is, Large Rep (Rep78, Rep68) transcripted and translated from the virus p5 promoter and Small Rep (Rep52, Rep40) transcripted and translated from the p19 promoter. The Large Rep and the Small Rep are changed in size by splicing. The REP protein encoded by the rep gene is involved in AAV replication, but on the other hand it inhibits replication of adenovirus and has cytotoxicity. Base sequences of the rep gene are represented by SEQ ID NO: 5 and SEQ ID NO: 6, but they are not limited to these sequences. Those whose sequences are partially deleted, substituted, or added to can also be included in the rep gene referred to in the present specification as long as they have substantially the same function and properties.

An antisense gene transferred into a cell of the present invention may be one or two or more antisense genes. In the case where two or more antisense genes are transferred into a cell, a plurality of copies of the same type of antisense gene may be transferred, or different types of antisense genes, that is, a plurality of antisense genes complementary to a plurality of genes encoding different types of polypeptides may be transferred. Furthermore, the base sequence of the antisense gene may be partially deleted, substituted, or added to as long as it has the property of suppressing expression of a gene encoding a polypeptide having cytotoxicity.

The present invention is explained further in detail below by reference to examples, but the present invention is not limited to these examples.

### [Examples]

### Example 1 Construction of pCAGS-r/anti-p5+p19/LN plasmid

A primer was designed in which BglII site was so designed that it contains a transcription initiation site of the p5 promoter of the rep gene (reps; 5';-GAAGATCTTCCATTTTGAAGCGGGAGGTTTGAACG-3"; (SEQ ID NO: 2); the underlining denotes the BglII site), and a primer was designed in which the BglII site was designed about 200 bp downstream of a transcription initiation site of the p19 promoter (repA; 5';-GAAGATCTGAATTCGCCGCATTGAAGGAGATGTATGAGG-3'; (SEQ ID NO: 3); the underlining denotes the BglII site).

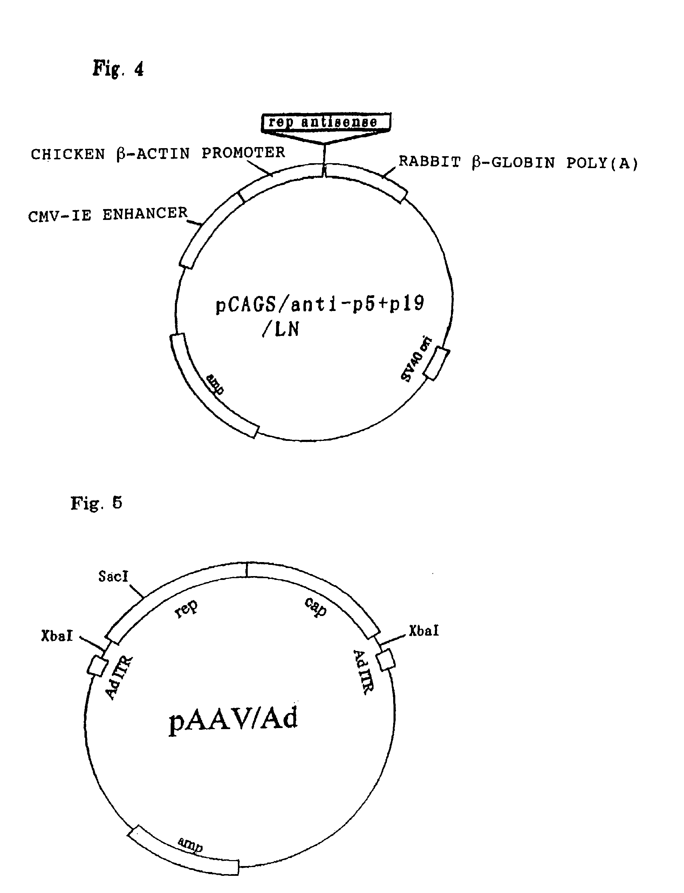

Part of the coding region of the rep gene was amplified by PCR (polymerase chain reaction) using as a template a pAAV/Ad plasmid containing the entire structural gene of AAV (Samulski, R. J., et al., J. Virol., 63, 3822-3828, 1989; FIG. 5; obtained from Samulski). The PCR conditions were: reaction at 94°C for 5 minutes, then 30 cycles of 94°C for 30 seconds, 61°C for 30 seconds, and 72°C for 1 minute, and further reaction at 72°C. The PCR product was subjected to electrophoresis using a 2% agarose gel and 1x TAE buffer according to a standard method, stained using ethidium bromide at a final concentration of 2 µg/ml, and examined under ultraviolet radiation, and it was confirmed that about 0.8 kb of an amplified product was obtained.

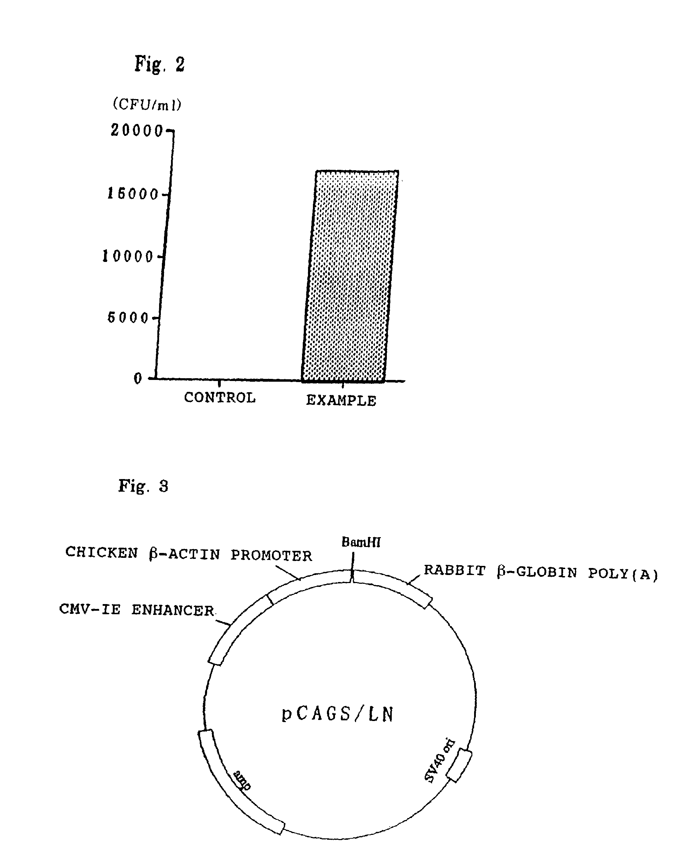

About 0.8 kb of this amplified product was collected from the gel and digested by BglII. Separately from this, after digesting pCAGS/LN (FIG. 3) by BamHI, the terminus was treated with BAP for dephosphorylation, and the aforementioned BglII-digested amplified product was subjected to subcloning. The clones obtained by the subcloning were subjected to a base sequence analysis, and a clone was selected in which the fragment was inserted in the direction in which the antisense rep gene was transcripted from the CAG promoter (pCAGS-r/anti-p5+p19/LN; FIG. 4).

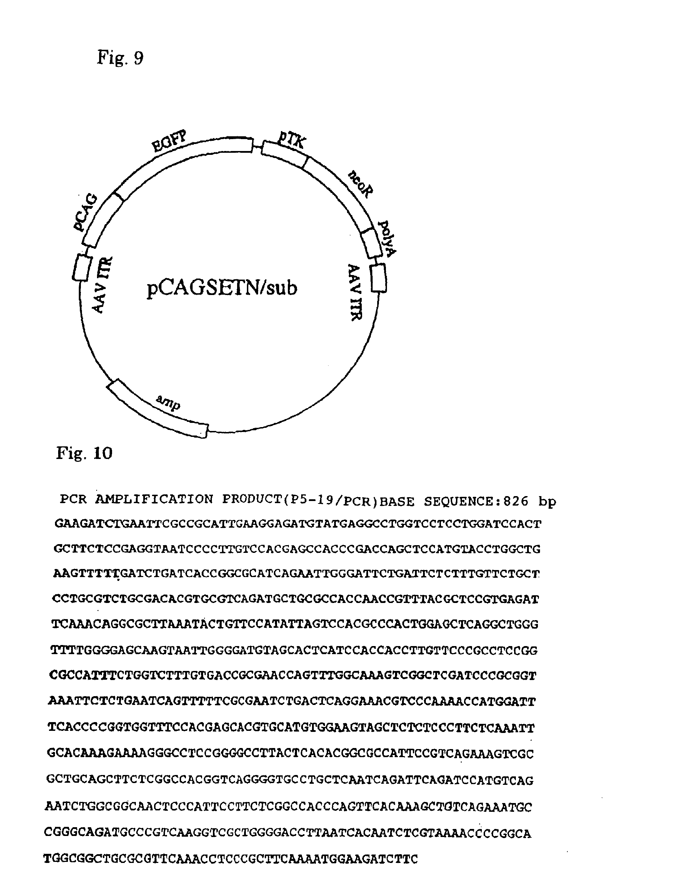

The base sequence of the amplified product is shown in FIG. 10.

The CAG promoter referred to here is a chimeric promoter formed from a CMV immediate-early enhancer/chicken β-actin promoter/β-globin poly(A).

In the figure, 'amp' denotes an ampicillin-resistant gene that can be used as a selection marker.

'CMV-IE enhancer' denotes an enhancer sequence of an early promoter derived from cytomegalovirus (CMV).

'SV40 ori' denotes a replication initiation site where the replication is carried out by an SV40 Large T antigen, and is derived from simian virus 40 (SV40).

'Rabbit β-globin poly(A)' denotes a poly(A) signal derived from rabbit β-globin gene.

### Example 2 Construction of pAdex1w/AAV plasmid

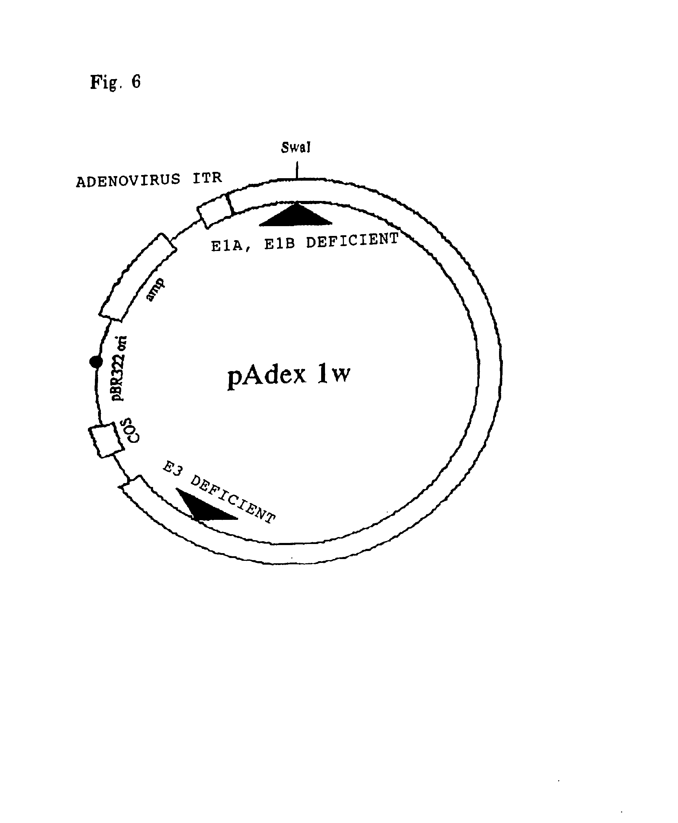

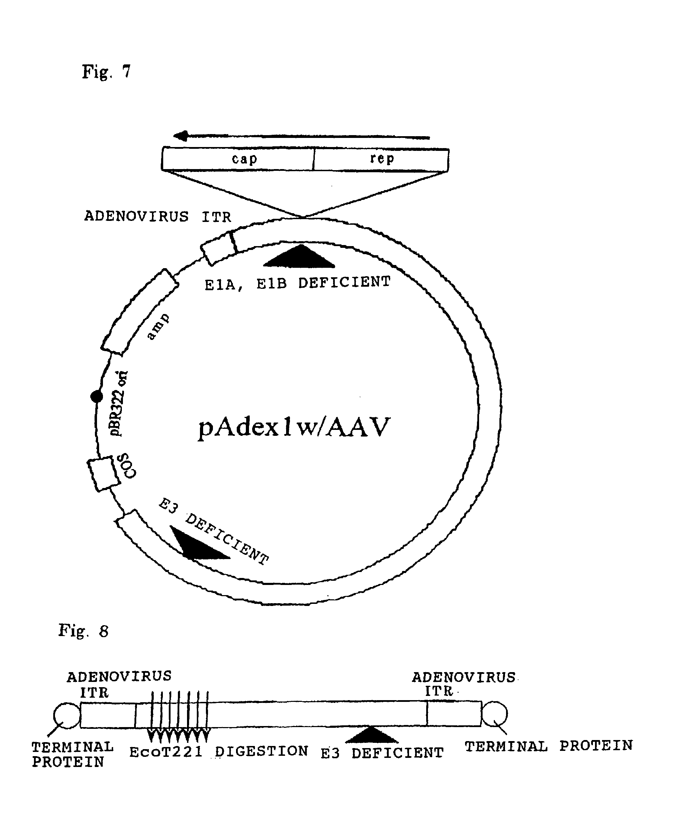

An approximately 4.3 kb XbaI fragment containing the rep gene and the cap gene of AAV was collected from pAAV/Ad (FIG. 5). This XbaI fragment was then subcloned at the SwaI site of the cosmid pAdexlw (Miyake, S. et al., Proc. Natl. Acad. Sci. USA., 93, 1320-1324, 1996; FIG. 6; obtained from Saito) to thus construct a pAdex1w/AAV cosmid (FIG. 7). Collection and subcloning of the gene fragment were carried out in the same manner as in Example 1.

In the figure, 'adenovirus ITR (AdITR)' denotes an ITR (Inverted Terminal Repeat) derived from an adenovirus type 5.

'rep' denotes an AAV type 2-derived rep gene.

'cap' denotes an AAV type 2-derived cap gene.

'COS' denotes a packaging recognition sequence derived from λ phage.

'pBR322 ori' denotes a plasmid replication initiation site within pBR322-derived E. coli.

### Example 3 Establishment of rep antisense expressing cell line

293 cells were used for establishing a rep antisense expressing cell line. The 293 cells were maintained in Dulbecco's modified Eagle's medium (DMEM; manufactured by Gibco Industries, Inc.) supplemented with 10% fetal bovine serum (Gibco Industries, Inc.) and an antibiotic. These cells were cultured to an approximately 70% confluent state in a 10 cm dish and transfected with the constructed pCAGS-r/anti-p5+p19/LN plasmid (FIG. 4) by the known calcium phosphate method (M. Kringler, Gene Transfer and Expression Protocol., A Laboratory Manua, Oxford University Press, 1990). The 293 cells thus transfected were incubated in a CO₂ incubator (culture conditions: cells were cultured using Dulbecco's modified Eagle's medium (DMEM; manufactured by Gibco Industries, Inc.) containing 10% fetal bovine serum (FBS, manufactured by Gibco Industries, Inc.) at 37°C and 5% oxygen concentration) for 4 hours, and then incubated for a further 2 days in fresh culture fluid.

In order to select only the transfectant, the cells were washed twice with a phosphate buffer (PBS(-), manufactured by Gibco Industries, Inc.), the cells were then detached from the culture dish by means of a trypsin-EDTA solution, and reinoculated in a fresh 10 cm culture dish. After confirming that the cells were attached to the culture dish, a G418 neomycin analogue (manufactured by Gibco Industries, Inc.) was added to the culture fluid at a final concentration of 1000 µg/ml. After incubating in a CO₂ incubator for 10 days, surviving cell clusters (colonies) were separated one by one and further incubated in fresh culture fluid to give a neomycin-resistant cell line (rep antisense expressing cells).

### Example 4 Confirmation of amount of rep antisense expressed

The total RNA was extracted from the rep antisense expressing cells obtained in Example 3 by the AGPC (Acid-Guanidium-Phenol-Chloroform) method and utilized. The total RNA (15 µg) thus prepared was subjected to electrophoresis in a 1% formaldehyde-agarose gel and fixed to a nitrocellulose filter (HybondN⁺: manufactured by Amersham plc). The pAAV/Ad (FIG. 5) was digested with XbaI and SacI, and an approximately 630 bp DNA fragment (SEQ ID NO: 4) specific to the p5 promoter region of the rep gene was collected.

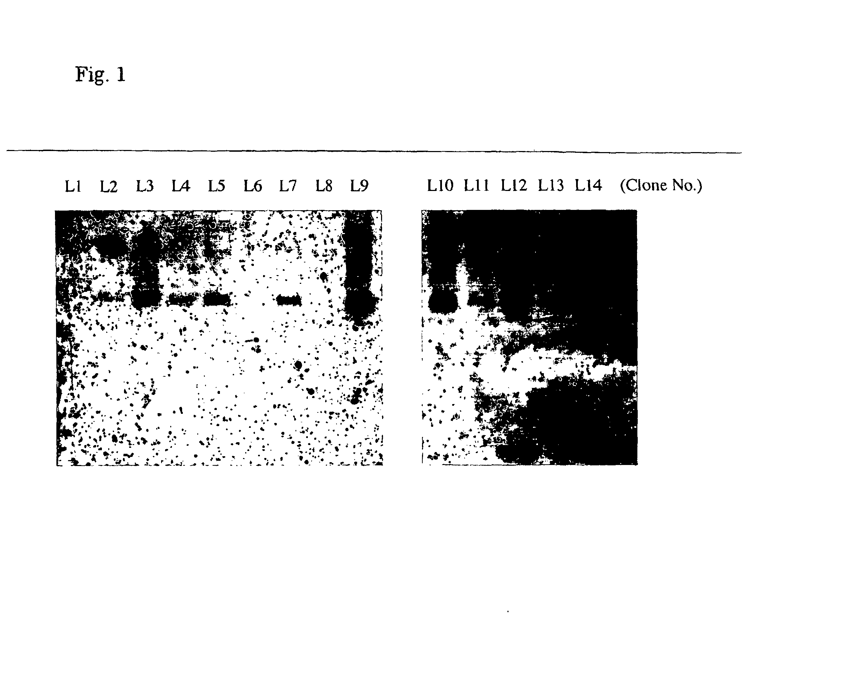

This fragment was labeled with ³²P to give a probe, which was used in Northern hybridization (42°C, 12 hours). A membrane was placed in a buffer containing 5x SSC, 50% formamide, 1x aqueous Denhart's solution, 0.2% SDS, 10% dextran sulfate, and 200 µg/ml thermally modified salmon sperm DNA, the radioisotope-labeled probe DNA was added, and hybridization was carried out at 65°C. The filter was subsequently washed in 2x SSC/0.5% SDS buffer at 55°C, and further washed in 0.1x SSC/0.5% SDS buffer at 55°C. It was then subjected to autoradiography on an X-ray film, thus confirming the expression of rep antisense in the neomycin-resistant cell line (FIG. 1). In particular, clone L9/293 (hereinafter called L9/293 cells) showed high rep antisense expression.

The L9/293 cell is a cell line that constantly expresses the antisense rep gene (SEQ ID NO: 1), and was deposited with the National Institute of Bioscience and Human-Technology under the Agency of Industrial Science and Technology on November 21, 2000 and accepted as Depository No. FERM BP-7377.

### Example 5 Preparation of rep-cap expressing adenovirus

The recombinant adenovirus vector used in this experiment was prepared according to the COS-TPC method (Miyake, S. et al., Proc. Natl. Acad. Sci. U.S.A., 93, 1320, 1996).

8 µg of the constructed pAdex1w/AAV cosmid and 5 µg of the EcoT22I-treated COS-TPC (FIG. 8) were transfected into the rep antisense expressing L9/293 cells (FIG. 1) established by the calcium phosphate method in Example 4. After the cells had been cultured at 37°C and 5% CO₂ for 12 hours, a 96-well plate was inoculated therewith, and they were cultured for further 10 to 15 days. The culture fluid of wells where the cells were dead was collected together with the dead cells, freeze thawed six times, and then centrifuged at 5,000 rpm for 5 minutes. The supernatant was stored as a first virus solution at -80°C. The L9/293 cells and HeLa cells were each infected with the first virus solution. Three days after the infection, no change was observed in the HeLa cells, and the culture fluid where the L9/293 cells were completely killed by the first virus solution was collected together with the dead cells of the cloned L9/293 cells. The culture fluid thus collected was freeze thawed six times, and then centrifuged at 5,000 rpm for 5 minutes. The supernatant was stored as a second virus solution at -80°C.

The cells used for preparing the second virus solution were collected, suspended in 1x TEN buffer (TEN: 50 mM Tris-HCl (pH 8.0), 10 mM EDTA, 100 mM NaCl), and homogenized. SDS (10%) at a final concentration of 0.1% and Proteinase K (manufactured by Merck, 20 mg/ml) at 0.1 mg/ml were added to the homogenized suspension, gently mixed by inversion, and incubated at 50°C for 1 hour. It was extracted twice with phenol/chloroform, the collected supernatant was further subjected twice to extraction with chloroform, and the supernatant was collected. DNA collected by ethanol precipitation was mixed with an appropriate amount of TE buffer (10 mM Tris-HCl (pH 8.0), 1 mM EDTA) to dissolve the DNA. This DNA was digested with EcoRI and then subjected to agarose gel electrophoresis, thus confirming the presence or absence of the adenovirus genome and the inserted gene.

The second virus solution of the clone selected by the above operation was used to infect L9/293 cells, the culture fluid where the cells were dead was collected together with the dead cells, freeze thawing was repeated six times, and this was followed by centrifuging at 3,000 rpm for 10 minutes. The supernatant was stored as a third virus solution at -80°C. The same procedure was carried out for scaling up and a fourth virus solution (hereinafter, called Ad/AAV solution) was finally prepared and stored at -80°C.

### Example 6 Preparation of recombinant AAV vector using Ad/AAV solution

An AAV vector was prepared using the Ad/AAV solution.

293 cells were cultured in a 10 cm dish using 10 ml of DMEM (10% FCS) to a 70% confluent state.

One ml of DMEM (2% FCS) to which 60 µl of the Ad/AAV solution prepared in Example 5 had been added were added to the dish, from which the medium had been removed, and incubated for 1 hour. After 8 ml of DMEM (10% FCS) was added, the 293 cells were transfected by the calcium phosphate method with 10 µg of pCAGSETN/sub vector plasmid (FIG. 9), which is a recombinant AAV vector containing, between the AAV-derived ITRs, an EGFP (which is an Aequorea coerulescens-derived green fluorescent protein and has the property of emitting green fluorescence under UV irradiation) gene that is induced by the CAG promoter, and a neomycin-resistant gene that is induced by the TK promoter (a promoter derived from a herpes simplex virus-thymidine kinase gene). Six hours later, the culture medium was removed, 6 ml of DMEM (10% FCS) was added, and culturing was carried out for 2 days.

The cells were collected together with the culture fluid, ruptured by repeated freeze thawing, and then centrifuged at 3,000 rpm for 10 minutes, and the supernatant was collected. It was heated at 56°C for 45 minutes so as to deactivate the adenovirus, and then centrifuged at 3,000 rpm for 10 minutes, and the supernatant (recombinant AAV vector solution) was collected.

### Example 7 Measurement of AAV vector titer

The titer of the recombinant AAV vector obtained in Example 6 was determined by a bioassay method using HeLa cells described below.

1 x 10⁵ HeLa cells were inoculated into a 6 well culture dish and allowed to stand overnight in a CO₂ incubator. The cells were washed twice with PBS (-), and 1 ml of the recombinant AAV vector solution was then added thereto. It was allowed to stand for 4 hours in the CO₂ incubator, then mixed with 3 ml of DMEM containing 10% FCS, and incubated in the CO₂ incubator for 2 days. After washing with PBS(-), the cells were detached from the culture dish by means of a trypsin-EDTA solution, and reinoculated in a fresh 10 cm culture dish. After confirming that the cells were attached to the culture dish, a G418 neomycin analogue (manufactured by Gibco Industries, Inc.) was added to the culture fluid at a final concentration of 1000 µg/ml. After incubating in the CO₂ incubator for 10 days, surviving cell clusters (colonies) were stained with crystal violet, and the number of colonies thus stained was counted (FIG. 2). As a control, the number of colonies was counted when untreated HeLa cells were treated with G418. From the results, it was found that the AAV vector could be prepared by using the rep antisense expressing cell line.

The above results show that, with regard to a process for producing an AAV vector comprising
(1) a step of inserting a rep gene having cytotoxicity into an adenovirus (helper virus) genome,
(2) a step of mass-propagating the helper virus, and
(3) a step of preparing an AAV vector using the helper virus, in step (2) the use of the cells into which the antisense gene of the present invention has been transferred can, without complicated operations, suppress the production of a Rep protein having cytotoxicity, thereby preventing the cells from dying and producing the helper virus.

### Industrial Applicability

By the use of the cells of the present invention a high titer virus vector solution is obtainable said cells having been transferred thereinto the antisense gene that expresses antisense RNA complementary to the entire sequence or part of the sequence of sense RNA that is expressed by a gene encoding a polypeptide having cytotoxicity.

## Claims

1. Cells to be used in producing a virus vector, the cells having been transferred thereinto one or two or more antisense genes that express an antisense RNA complementary to the entire sequence or a partial sequence of a sense RNA that is expressed by a gene encoding a polypeptide having cytotoxicity.

2. The cells according to Claim 1, wherein the gene encoding the polypeptide having cytotoxicity is a virus vector-derived gene.

3. The cells according to Claim 2, wherein the virus vector-derived gene is an adeno-associated virus vector-derived gene.

4. The cells according to Claim 3, wherein the adeno-associated virus vector-derived gene is a rep gene.

5. The cells according to Claim 1, wherein the antisense gene is an antisense gene that expresses an antisense RNA complementary to the sequence represented by SEQ ID NO: 1 and/or a sequence that is obtained by partially deleting, substituting, or adding to said sequence.

6. The cells according to Claim 5, wherein the cells are cells designated by Depository No. FERM BP-7377.

7. The cells according to Claim 1, wherein the polypeptide having cytotoxicity is a polypeptide that inhibits growth of a helper virus.

8. The cells according to Claim 7, wherein the helper virus is an adenovirus.

9. A process for producing cells to be used in producing a virus vector, comprising transferring one or two or more antisense genes that express an antisense RNA complementary to the entire sequence or a partial sequence of a sense RNA that is expressed by a gene encoding a polypeptide having cytotoxicity.

10. The process according to Claim 9, wherein the gene encoding the polypeptide having cytotoxicity is a virus vector-derived gene.

11. The process according to Claim 10, wherein the virus vector-derived gene is an adeno-associated virus vector-derived gene.

12. The process according to Claim 11, wherein the adeno-associated virus vector-derived gene is a rep gene.

13. The process according to Claim 9, wherein the antisense gene is an antisense gene that expresses an antisense RNA complementary to a sequence represented by SEQ ID NO: 1 and/or a sequence that is obtained by partially deleting, substituting, or adding to said sequence.

14. The process according to Claim 13, wherein the cells are cells designated by Depository No. FERM BP-7377.

15. The process according to Claim 9, wherein the polypeptide having cytotoxicity is a polypeptide that inhibits growth of a helper virus.

16. The process according to Claim 15, wherein the helper virus is an adenovirus.

17. A process for producing a virus vector with the use of the cells according to any one of Claims 1 to 8, comprising:
a step of obtaining a helper virus that expresses a gene derived from the virus vector; and
a step of transfecting cells into which no antisense gene has been transferred with the helper virus that expresses the gene derived from the virus vector and a virus vector plasmid.
